Europäisches Patentamt

European Patent Office

Office européen des brevets

(11) Veröffentlichungsnummer: **0 088 153**
**A2**

(12) **EUROPÄISCHE PATENTANMELDUNG**

(21) Anmeldenummer: **82107765.8**

(22) Anmeldetag: **24.08.82**

(51) Int. Cl.³: **H 05 G  1/60,** H 05 G  1/64,
A 61 B  6/02

(30) Priorität: **04.03.82 DE 3207816**

(43) Veröffentlichungstag der Anmeldung: **14.09.83**
**Patentblatt 83/37**

(84) Benannte Vertragsstaaten: **DE FR GB**

(71) Anmelder: **SIEMENS AKTIENGESELLSCHAFT, Berlin
und München Wittelsbacherplatz 2,
D-8000 München 2 (DE)**

(72) Erfinder: **Sklebitz, Hartmut, Würzburger Ring 45,
D-8520 Erlangen (DE)**
Erfinder: **Maass, Wolfgang, Haunritzer Weg 2,
D-8500 Nürnberg (DE)**

(54) **Röntgendiagnostikeinrichtung für Röntgenschichtbilder.**

(57) Die Erfindung betrifft eine Röntgendiagnostikeinrichtung für Röntgenschichtbilder mit wenigstens einer Röntgenstrahlenquelle (1) zur Erzeugung eines Röntgenstrahlenbündels, mit einem Steuergenerator (9) zur Bewegung des Röntgenstrahlenbündels, mit einer Patientenliege (2), mit einem Röntgenbildverstärker (3), der von Ablenkspulen (7) umgeben ist, die mit einer an dem Steuergenerator (9) angeschlossenen Ablenkvorrichtung (8) für die synchrone Bewegung des Elektronenbildes im Röntgenbildverstärker (3) verbunden sind, und mit einer am Ausgang des Röntgenbildverstärkers (3) angekoppelten Fernsehkette (4 bis 6), bei der die Ablenkspulen (7) aus n in einer Ebene um 369°/n gegeneinander versetzten Spulenpaaren besteht, wobei n wenigstens drei beträgt. Die Ablenkvorrichtung (8) ist so ausgebildet, daß der Ablenkstrom nur in jeweils zwei der n Spulenpaare fließt.

SIEMENS AKTIENGESELLSCHAFT
Berlin und München

0088153

Unser Zeichen
VPA 82 P 3712 E

## Röntgendiagnostikeinrichtung für Röntgenschichtbilder

Die Erfindung betrifft eine Röntgendiagnostikeinrichtung für Röntgenschichtbilder mit wenigstens einer Röntgenstrahlenquelle zur Erzeugung eines Röntgenstrahlenbündels, mit einem Steuergenerator zur Bewegung des Röntgenstrahlenbündels im Sinne der Durchstrahlung des Patienten aus verschiedenen Richtungen, mit einer Patientenliege, mit einem Röntgenbildverstärker, der von Ablenkspulen umgeben ist, die mit einer an dem Steuergenerator angeschlossenen Ablenkvorrichtung für die synchrone Bewegung des Elektronenbildes im Röntgenbildverstärker verbunden sind, und mit einer am Ausgang des Röntgenbildverstärkers angekoppelten Fernsehkette. Mit dieser Röntgendiagnostikeinrichtung erhält man Röntgenschichtbilder in Längsrichtung des Patienten.

In der DE-OS 27 12 320 ist eine solche Röntgendiagnostikeinrichtung beschrieben, bei der durch eine synchrone Bewegung der Röntgenstrahlung und des Ausgangsbildes des Röntgenbildverstärkers eine Körperschicht scharf abgebildet wird, während alle anderen, nicht in dieser Schicht liegenden Körperteile durch Unschärfe unterdrückt werden. Die gewünschte Schicht kann durch die Veränderung der Ablenkung des Elektronenbildes im Röntgenbildverstärker ausgewählt werden. Zwei um den Röntgenbildverstärker angeordnete Spulenpaare erzeugen ein Magnetfeld, das die Ablenkung des Elektronenbildes des Röntgenbildverstärkers bewirkt.

Die um den Röntgenbildverstärker angeordneten beiden

Gse 5 Ler / 02.03.1982

Spulenpaare müssen entsprechend der Auslenkung des Elektronenbildes im Röntgenbildverstärker, die von der geforderten maximalen Schichthöhe abhängt, unter Umständen mit hohen Strömen beaufschlagt werden, so daß Verzerrungen des Magnetfeldes auftreten können, die die Auflösung des Schichtbildes beeinträchtigen. Diese Verzerrungen begrenzen den möglichen Schichthöhenbereich. Weiterhin werden an den Röntgenbildverstärker dabei hohe Anforderungen gestellt. Selbst geringe differentielle Verzeichnungen, die insbesondere zum Rande des Röntgenbildverstärkers hin auftreten, schränken den nutzbaren Bereich des Eingangsbildes des Röntgenbildverstärkers ein, wodurch die maximal mögliche Schichthöhe weiter reduziert wird. Weiterhin treten bei der Verwendung von beispielsweise rechteckförmigen Strömen für die Ablenkspulen kometenschweifförmige Bildverzeichnungen auf, die ebenfalls die Auflösung des Röntgenschichtbildes beeinträchtigen.

Die Erfindung geht von der Aufgabe aus, eine Röntgendiagnostikeinrichtung der eingangs genannten Art zu schaffen, die eine gleichbleibende Ablenkung bei reduzierten Strömen ermöglicht, so daß Verzerrungen verhindert und Verzeichnungen kompensiert werden.

Die Aufgabe ist erfindungsgemäß dadurch gelöst, daß die Ablenkspulen aus n in einer Ebene um $360^{\circ}/n$ gegeneinander versetzten Spulenpaaren bestehen, wobei n wenigstens drei beträgt, und daß die Ablenkvorrichtung so ausgebildet ist, daß der Ablenkstrom nur in jeweils zwei der n Spulenpaare fließt. Durch diese Anordnung wird erreicht, daß zur Erzeugung eines Magnetfeldes der durch die Spulen fließende Strom immer auf zwei Spulen verteilt wird, so daß durch die einzelnen Spulen ein geringerer Strom als für die Ablenkung erforderlich fließt.

Die differentiellen Verzeichnungen des Röntgenbildverstärkers lassen sich besonders gut kompensieren, wenn die Ablenkvorrichtung Spannungen erzeugt, die einen treppenförmigen Verlauf aufweisen, wobei jede Treppenstufe einer Röntgenstrahlenquelle zugeordnet ist, wenn die Ablenkvorrichtung mit einer Generatorschaltung, welche für die Übergänge von Stufe zu Stufe eine für jede Stufe der Treppenfunktion durch Einstellmittel separat einstellbare e-Funktion erzeugt, und mit einer Überlagerungsstufe versehen ist, in der die treppenförmige Spannung und die e-Funktion überlagert werden, und wenn der treppenförmige Spannungsverlauf sich von einem Anfangswert, der dem Spannungswert der vorhergehenden Treppenstufe entspricht, einem der aktuellen Treppenstufe entsprechenden Endwert in Form der e-Funktion annähert. Ein einfacher Aufbau der Ablenkvorrichtung wird durch die in den weiteren Unteransprüchen angegebenen Maßnahmen erreicht.

Die Erfindung wird nachfolgend anhand eines in der Zeichnung dargestellten Ausführungsbeispieles näher erläutert. Es zeigen:

Fig. 1 eine Röntgendiagnostikeinrichtung gemäß der Erfindung, und

Fig. 2 ein Blockschaltbild einer in Figur 1 dargestellten Ablenkvorrichtung.

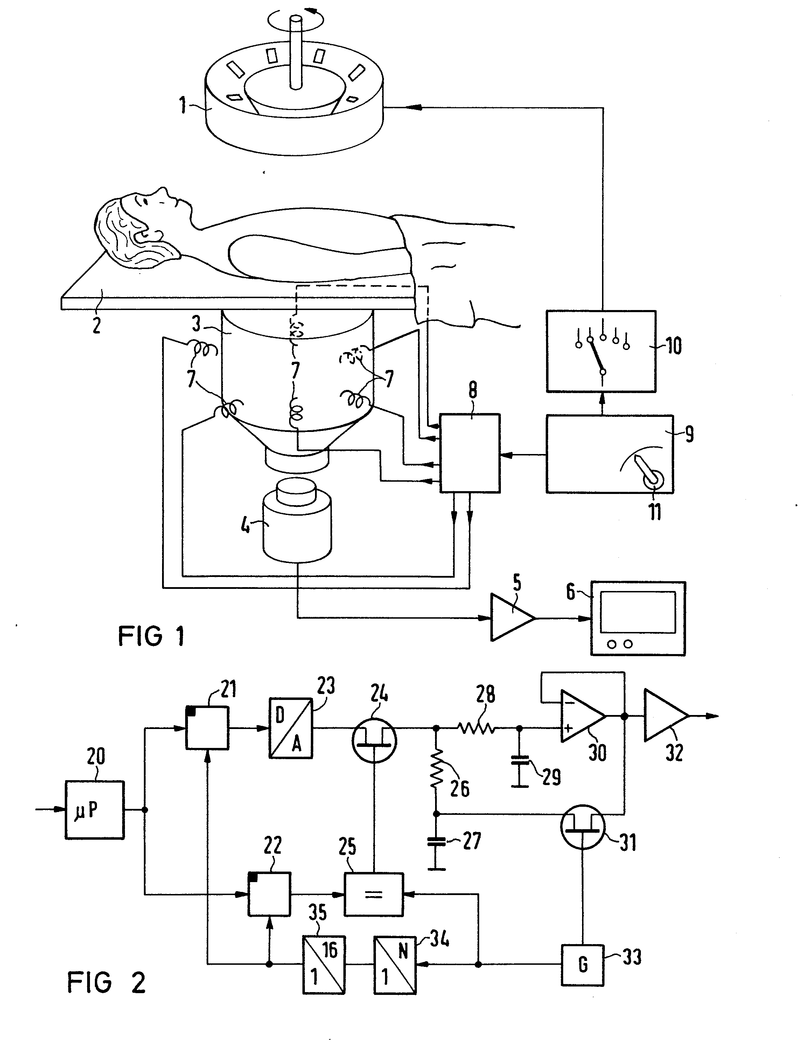

In der Figur 1 ist eine Röntgendiagnostikeinrichtung mit einer Röntgenröhre 1 dargestellt, die eine kreisringförmige Anordnung von gittergesteuerten Kathoden aufweist, die um eine Drehanode angeordnet sind. Eine Röntgenröhre dieser Art ist in der deutschen Patentanmeldung P 31 13 368.1 beschrieben. Der Hochspannungstransformator für die Röntgenröhre 1 ist nicht dargestellt. Die Röntgenröhre 1 erzeugt Röntgenstrahlenbündel, die einen auf einer Patientenliege 2 liegenden Patienten durchdringen und auf den Eingangsleuchtschirm eines Röntgenbildverstärkers 3 Strahlenbilder werfen. Das Ausgangssignal des Röntgenbildverstärkers 3 wird durch eine Fernsehkamera 4 aufgenommen, deren Ausgangssignal in einem Videoverstärker 5 verstärkt und auf einem Monitor 6 wiedergegeben wird.

Die für die Schichttechnik erforderliche Ablenkung des Elektronenbildes im Röntgenbildverstärker 3 wird magnetisch mittels dreier Paare von Ablenkspulen 7 erreicht. Die Ansteuerung der Ablenkspulen 7 erfolgt durch eine Ablenkvorrichtung 8, die von einem Steuergenerator 9 synchronisiert wird. Der Steuergenerator 9 bewirkt synchron mit der Bildverschiebung im Röntgenbildverstärker 3 über eine Steuervorrichtung 10 die schrittweise Einschaltung der Gitter der Röntgenröhre 1. Ein Stellmittel 11, das als Potentiometer ausgeführt sein kann, ist an dem Steuergenerator 9 angebracht und bestimmt die maximale Ablenkamplitude und dadurch die Höhe der betrachteten Körperschicht.

Anstelle der Röntgenröhre 1 können auch mehrere einzelne Röntgenröhren vorgesehen sein, die kreisförmig, linear oder beliebig verteilt angeordnet sein können und synchron mit der Ablenkung des Röntgenbildverstärkers 3 eingeschaltet werden. Es kann aber auch ein Röntgenbrennfleck verwendet werden, der mechanisch bewegt wird.

Die Körperschicht, deren Details auf dem Monitor 6 scharf dargestellt werden, kann außer durch Intensitätsänderung der magnetischen Bildablenkung auch durch
Einstellung des Abstandes zwischen der Röntgenröhre 1
und der Patientenliege 2 sowie dem Röntgenbildverstärker 3 gewählt werden.

In der Figur 2 ist ein Mikroprozessor 20 dargestellt,
dessen Datenausgang mit den Eingängen zweier Speicher
(RAM) 21 und 22 verbunden ist. Der Ausgang des Speichers 21 ist an einem Digital/Analog-Wandler 23 angeschlossen, der mit dem Sourceanschluß eines Feldeffekttransistors (FET) 24 verbunden ist. Der eine Eingang
eines Komparators 25 ist mit dem Speicher 22 verbunden,
und der Ausgang des Komparators 25 ist an dem Gateanschluß des FET 24 angeschlossen. Mit dem Drainanschluß
des Feldeffekttransistors 24 sind zwei Widerstände 26
und 28 verbunden, deren andere Anschlüsse über Kondensatoren 27 und 29 auf Masse gelegt sind. Der Verbindungspunkt des Kondensators 29 und des Widerstandes 28
ist auf den nichtinvertierenden Eingang eines Operationsverstärker 30 gelegt. Der Ausgang des Operationsverstärkers 30 ist mit seinem invertierenden Eingang
verbunden. Ein Feldeffekttransistor 31 verbindet den
Ausgang des Operationsverstärkers 30 und den Verbindungspunkt des Widerstandes 26 und des Kondensators 27.
Die Ausgangsspannung des Operationsverstärkers 30, der
als Impedanzwandler geschaltet ist, wird durch einen
Endverstärker 32 linear in Stromwerte umgesetzt, die
einer Ablenkspule 7 zugeführt werden. Für jede der Ablenkspulen 7 muß eine Ansteuerungsschaltung 21 bis 32
vorgesehen sein. Sie sind aber, damit die Darstellung
nicht unübersichtlich wird, in der Figur 1 nicht abgebildet. Ein Generator 33 erzeugt die Schaltimpulse für
den FET 31 und Taktimpulse, die auf den Komparator 25
und auf einen ersten Teiler 34 geführt sind. Dieser
erste Teiler 34 teilt die Taktimpulse entsprechend der Anzahl der Spulenpaare. Das Ausgangssignal des ersten Teilers 34 wird in einem zweiten Teiler 35 beispielsweise durch 16 geteilt. Der Ausgang dieses Teilers 35 ist mit den Takteingängen der Speicher 21 und 22 verbunden.

Vom Stellmittel 11 des Steuergenerators 9 wird ein Signal dem Mikroprozessor 20 zugeführt, das einer eingestellten Schichthöhe entspricht. Die für diese bestimmte Schichthöhe benötigten Ablenkdaten werden aus dem Mikroprozessor in den Speicher 21 übertragen. Mit dem Takt für die Einschaltung der Röntgenstrahlenquellen werden diese Ablenkdaten ausgelesen. Am Ausgang des D/A-Wandlers 23 stehen analoge Ablenkamplituden zur Steuerung der Ablenkströme zur Verfügung, die den treppenförmigen Verlauf bilden. Für die Bestimmung der Zeitkonstanten der e-Funktion werden gleichzeitig Daten in den Speicher 22 übertragen, die ebenfalls im Takt der Röntgenstrahlenquellen ausgelesen werden. Der Komparator 25 liefert durch Vergleich der Taktimpulse mit den jeweiligen Daten Schaltimpulse vorbestimmter Länge, so daß die am Ausgang des D/A-Wandlers 23 anliegende Spannung durch den FET 24 durchgeschaltet wird. Die Länge der Schaltimpulse bestimmt die Aufladegeschwindigkeit des Kondensators 27. Da die Vergleichsfrequenz in diesem Beispiel mit drei Spulenpaaren 48 mal höher liegt als die Schaltfrequenz der Röntgenstrahlenquellen, erhält man am Kondensator 27 praktisch eine e-Funktion. Der Widerstand 28 und der Kondensator 29 stellen einen Tiefpaß dar, der die Rückstände der hohen Schaltfrequenz des FET unterdrückt. Die Zeitkonstante des Tiefpasses 28, 29 ist erheblich kleiner als die des Widerstandes 26 und des Kondensators 27. Am Ausgang der Verstärker 30 und 32 liegt somit eine Spannung mit einem Verlauf einer e-Funktion. Nach beendigter Aufnahme wird die zugehörige Röntgenstrahlenquelle gesperrt und vor dem Umschalten zur nächsten Treppenstufe erhält der FET 31 von dem Generator 33 einen kurzen Schaltimpuls, so daß der dem Endwert des Stromwertes entsprechende Spannungswert der jetzt beendeten Treppenstufe in den Kondensator 27 gespeichert wird. Jetzt wird eine neue Adresse an die Speicher 21 und 22 angelegt, so daß die nächste Treppenstufe erzeugt werden kann. Gleichzeitig wird die dieser Treppenstufe entsprechende Röntgenstrahlenquelle eingeschaltet. Wie bereits beschrieben, wird der Kondensator 27 entsprechend der eingestellten e-Funktion wieder umgeladen.

Durch solche Maßnahmen lassen sich zusätzlich Auflösungsminderungen durch Geräteverwindung beim Kippen des Gerätes, durch variable Erdmagnetismus-Einflüsse beim Kippen und durch unvollständige Zentrierung von Strahler und Röntgenbildverstärker vermeiden.

Für die verschiedenen Schichthöhen sind die Daten für die Ablenkamplituden in einem Speicherbereich des Mikroprozessors abgelegt. Werden Zwischenstufen benötigt, so können diese durch Interpolation im Mikroprozessor gebildet werden.

Für die Justierung der Ablenkströme können die gespeicherten Daten durch nicht dargestellte Einstellmittel korrigiert werden. Dies kann aber auch durch eine Umschaltung der Adressen durch den Mikroprozessor erfolgen.

Die Einstellung wird vorteilhaft mit Hilfe einer kleinen Stahlkugel vorgenommen. Bei etwa maximaler Schichthöhe wird die Einstellung für jede der Röntgenstrahlenquellen auf jeder Ablenkachse so lange variiert, bis
die Stahlkugel frei von kometenschweifförmigen Bildverzeichnungen abgebildet wird.


2 Figuren
5 Patentansprüche

0088153

Patentansprüche

1. Röntgendiagnostikeinrichtung für Röntgenschichtbilder mit wenigstens einer Röntgenstrahlenquelle (1) zur Erzeugung eines Röntgenstrahlenbündels, mit einem Steuergenerator (9) zur Bewegung des Röntgenstrahlenbündels im Sinne der Durchstrahlung des Patienten aus verschiedenen Richtungen, mit einer Patientenliege (2), mit einem Röntgenbildverstärker (3), der von Ablenkspulen (7) umgeben ist, die mit einer an dem Steuergenerator (9) angeschlossenen Ablenkvorrichtung (8) für die synchrone Bewegung des Elektronenbildes im Röntgenbildverstärker (3) verbunden sind, und mit einer am Ausgang des Röntgenbildverstärkers (3) angekoppelten Fernsehkette (4 bis 6), d a d u r c h   g e k e n n - z e i c h n e t ,   daß die Ablenkspulen (7) aus n in einer Ebene von $360°/n$ gegeneinander versetzten Spulenpaaren besteht, wobei n wenigstens drei beträgt, und daß die Ablenkvorrichtung (8) so ausgebildet ist, daß der Ablenkstrom nur in jeweils zwei der n Spulenpaare fließt.

2. Röntgendiagnostikeinrichtung nach Anspruch 1, d a d u r c h   g e k e n n z e i c h n e t ,   daß die Ablenkvorrichtung (8) Spannungen erzeugt, die einen treppenförmigen Verlauf aufweisen, wobei jede Treppenstufe einer Röntgenstrahlenquelle (1) zugeordnet ist, daß die Ablenkvorrichtung (8) mit einer Generatorschaltung (22, 24 bis 27), welche für die Übergänge von Stufe zu Stufe eine für jede Stufe der Treppenfunktion durch Einstellmittel separat einstellbare e-Funktion erzeugt, und mit einer Überlagerungsstufe (23 bis 30) versehen ist, in der die treppenförmige Spannung und die e-Funktion überlagert werden.

3. Röntgendiagnostikeinrichtung nach Anspruch 2,
d a d u r c h   g e k e n n z e i c h n e t ,   daß
der treppenförmige Spannungsverlauf sich von einem Anfangswert, der dem Spannungswert der vorhergehenden
Treppenstufe entspricht, einem der aktuellen Treppenkurve entsprechenden Endwert in Form der e-Funktion
annähert.

4. Röntgendiagnostikeinrichtung nach Anspruch 2 oder 3,
d a d u r c h   g e k e n n z e i c h n e t ,   daß
die Generatorschaltung derart ausgebildet ist, daß sie
für die einzelnen Treppenstufen eine annähernd gleichbleibende e-Funktion erzeugt, und daß eine Multiplikationsstufe der Generatorschaltung vorgeschaltet ist,
in der die e-Funktion mit einem durch für jede Treppenstufe separate Einstellmittel veränderbaren Faktor
multipliziert wird.

5. Röntgendiagnostikeinrichtung nach einem der Ansprüche 1 bis 3,   d a d u r c h   g e k e n n z e i c h -
n e t ,   daß ein Mikroprozessor (20) und ein Generator (33) mit einem Teiler (34, 35) vorgesehen sind, daß
für jede der 2 x  n Ablenkspulen der Mikroprozessor mit
den Dateneingängen von Speichern (21, 22) verbunden ist,
daß ein Speicher (21) über einen D/A-Wandler (23) mit
einem Schalter (24) verbunden ist, der an zwei parallelen R-C-Gliedern (26 bis 29) mit unterschiedlichen
Zeitkonstanten angeschlossen ist, daß der Ausgang des
R-C-Gliedes (28, 29) mit kleiner Zeitkonstante über
einen Impedanzwandler (30) und einen Spannungs-Stromwandler (32) mit einer der Ablenkspulen (7) verbunden
ist, daß ein Schalter (31) den Ausgang des Impedanzwandlers (30) mit dem R-C-Glied (26, 27) mit größerer
Zeitkonstante verbindet, daß der Generator (33) den
Schalter (31) und einen Komparator (25) direkt und die Speicher (21, 22) über den Teiler (34, 35) ansteuert, und daß der Komparator (25) an dem Speicher (22) angeschlossen ist und in Abhängigkeit von seinen Eingangssignalen dem Schalter (24) einen Schaltimpuls liefert.

FIG 1

FIG 2